# EUROPEAN PATENT APPLICATION

(11) **EP 2 818 476 A1**
(43) Date of publication of application: **31.12.2014**
(21) Application number: 13751650.6
(22) Date of filing: 22.02.2013
(51) Int. Cl.: C07H 15/18, C07H 15/26, C07H 15/00, C07H 9/00, C07H 9/02, C07H 9/04, C07D 493/00, C07D 493/02, C07D 493/04

(54) **METHOD FOR PREPARING 3-O-BENZYL-1,2-O-ISOPROPYLIDENE- -L-FURAN IDOSE**

(30) Priority: 23.02.2012 CN 201210050766
(71) Applicant: Zhejiang Hisun Pharmaceutical Co. Ltd., Zhejiang 318000 (CN)
(72) Inventor: GUO, Yanghui, Zhejiang 318000 (CN); WEI, Hegeng, Zhejiang 318000 (CN); ZHOU, Junhui, Zhejiang 318000 (CN); WU, Yingqiu, Zhejiang 318000 (CN); ZHANG, Yue, Zhejiang 318000 (CN); BAI, Hua, Zhejiang 318000 (CN); HE, Liang, Zhejiang 318000 (CN); DING, Yili, Zhejiang 318000 (CN)
(74) Representative: Kador & Partner
(86) International application number: PCT/CN2013/071786
(87) International publication number: WO 2013/123896

(57) **Abstract**

Provided is a method for preparing 3-O-benzyl-1,2-O-isopropylidene-α-L-idofuranose, which comprises: (1) protecting hydroxyl of 3-O-benzyl-1,2-O-isopropylidene-α-D-glucofuranose (III) by benzoyl and methylsulfonyl to obtain 6-O-benzoyl-3-O-benzyl-1,2-O-isopropylidene-5-O-methylsulfonyl-α-D-glucofuranose (V); (2) subjecting compound (V) to a cyclization reaction under an alkaline condition to obtain 5,6-epoxy-3-O-benzyl-1,2-O-isopropylidene-α-L-idofuranose (VI); and (3) subjecting compound (VI) to a ring-opening reaction to obtain 3-O-benzyl-1,2-O-isopropylidene-α-L-idofuranose.

## Description

### Field of the Invention

The present invention relates to the pharmaceutical field. Specifically, it relates to a novel method for preparing 3-O-benzyl-1,2-O-isopropylidene-α-L-idofuranose.

### Background of the Invention

The mucopolysaccharide in mammals comprises heparin, heparin sulfate, and dermatan sulfate etc., which plays an important role in various physiological processes and L-iduronic acid unit is the key component thereof. It is always a difficulty in synthesizing fragments of an idose in the sugar chemistry, which has restricted the development of research on the artificial synthesis of heparin-like molecules, and has become one of the bottlenecks in the synthesis of the drug Fondaparinux sodium.

The available methods for preparing derivatives of L-idose include: inversion of configuration of a 5-hydroxyl group by hydroboration-oxidation reaction on a 5,6-exocyclic double bond of a glucose (Hinou, Hiroshi; Kurosawa, Hidehiro; Matsuoka, Koji; Terunuma, Daiyo; Kuzuhara, Hiroyoshi; Tetrahedron Lett. 1999, 40, 1501; Hung, S. C.; Thopate, S. R.; Chi, F. C.; Chang, S. W.; Lee, J. C.; Wang, C. C.; Wen, Y S. J. Am. Chem. Soc. 2001, 123, 3153); inversion of configuration by isomerization through a free radical reaction of 5-bromo-glucuronic acid (Chiba, T.; Sinay, P. Carbohydr. Res. 1986, 151, 379); constructing chirality at 5-position by using anions such as cyano, (PhS)₃CLi, etc. to conduct an asymmetric addition to an aldehyde (Lubineau, A.; Gavard, O.; Alais, J.; Bonnaffe, D. Tetrahedron Lett. 2000, 41, 307; Jarosz, S. Carbohydr. Res. 1987, 166, 211; Kapeller, D. C; Hammerschmidt, F. Tetrahedron 2010, 66, 591); inversion of configuration of the 5-hydroxyl group by nucleophilic substitution on a trifluoromethanesulfonate or a methane sulfonate of glucose or glucuronic acid (Orgueira, H. A.; Bartolozzi, A.; Schell, P.; Litjens, R. E. J. N.; Palmacci, E. R.; Seeberger, P. H. Chem. Eur. J. 2003, 9, 140; Ke, W.; Whitfield, D. M.; Gill, M.; Larocque, S.; Yu, S. Tetrahedron Lett. 2003, 44, 7767; Tadano, K., Idogaki, Y., Yamada, H., Suami, T. J. Org. Chem. 1987, 52, 1201; Barroca, N.; Jacquinet, J. C. Carbohydr. Res. 2000, 329, 667). These methods have low yields, long routes, difficulties in operating, high costs, and the subsequent distinguishing operations to protective groups are relatively difficult. The available methods further include inversion of configuration of the 5-hydroxyl group by utilizing an acidic ring-opening of the epoxy group (Van Boeckel, C. A. A.; Beetz, T.; Vos, J. N.; De Jong, A. J. M.; Van Aelst, S. F; Van den Bosch, R. H.; Mertens, J. M. R.; Van der Vlugt, F. A. J. Carbohydr. Chem. 1985, 4, 293), but no effective, convenient, suitable method for a large scale industrial production on the ring-opening epoxy after inversion of configuration has been found.

### Summary of the Invention

The present invention provides a method for preparing 3-O-benzyl-1,2-O-isopropylidene-α-L-idofuranose VII. The method comprises: conducting a selective ring-opening of compound VI under a basic condition to obtain compound VII:

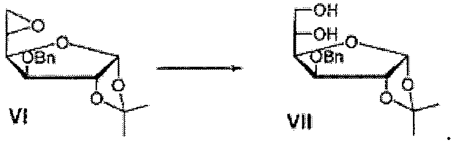

In some embodiments, as for the ring-opening reaction of compound VI under a basic condition to obtain compound VII, the reaction temperature may be from -30 °C to +100 °C, preferably from 0 °C to +100 °C, more preferably from +60 °C to +80 °C. The base may preferably be an inorganic base. The inorganic base may preferably be potassium hydroxide, sodium hydroxide, lithium hydroxide, magnesium hydroxide, calcium hydroxide, barium hydroxide. The reaction solvent may preferably be water, methanol, ethanol, propanol, t-butanol, tetrahydrofuran, dioxane, acetonitrile, N,N-dimethyl formamide, dimethyl sulfoxide, or any mixtures thereof. A crude product may be refined by recrystallization.

In further embodiments, compound VI may be prepared by the following method: cyclization of compound V is conducted under a basic condition, followed by obtaining 5,6-epoxy compound VI with inversion of configuration at 5-position:

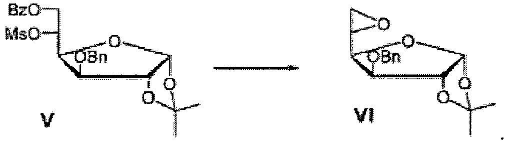

In some embodiments, during the reaction of compound V under a basic condition of forming 5,6-epoxy compound VI with inversion of configuration at 5-position, the reaction temperature may be from -30 °C to +60 °C, preferably from 0 °C to +40 °C, more preferably from +20 °C to +30 °C. The amount of base may be from 1 to 10 times of that of compound V (in molar ratio). The base may preferably be inorganic bases, and organic bases, wherein the inorganic base may preferably be potassium hydroxide, sodium hydroxide, lithium hydroxide, magnesium hydroxide, calcium hydroxide, barium hydroxide, as well as wherein the organic base may preferably be pyridine, triethylamine, sodium C₁-C₄ lower alcoholate or potassium C₁-C₄ lower alcoholate, wherein the preferred sodium C₁-C₄ lower alcoholate or potassium C₁-C₄ lower alcoholate is sodium methoxide, sodium ethoxide, sodium tert-butoxide, potassium tert-butoxide. The reaction solvent may preferably be water, methanol, ethanol, propanol, t-butanol, tetrahydrofuran, dioxane, acetonitrile, N,N-dimethyl formamide, dimethyl sulfoxide, dichloromethane, or any mixtures thereof. The reaction product may be conducted into the next step directly without any post-treatment, or the crude product after post-treatments may be used directly in the next step. In some embodiments, the reaction can be conducted with the above-mentioned ring-opening reaction within one reaction system.

In further embodiments, compound V may be prepared by the following method: by taking advantage of steric hindrance, 6-hydroxyl of compound III was firstly protected by a benzoyl group under a basic condition to obtain compound IV, and then 5-hydroxyl of compound IV was protected by methanesulfonyl group under a basic condition to obtain compound V:

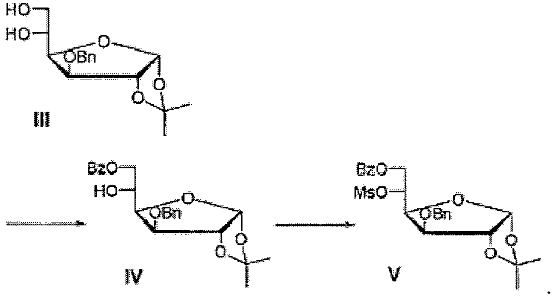

In some embodiments, in the reaction of selectively protecting 6-hydroxyl of compound III with a benzoyl group under a basic condition to obtain compound IV, the reaction temperature may be from -30 °C to +60 °C, and the amount of benzoyl chloride may be from 0.6 to 2.0 times of that of compound V (in molar ratio), which was added in batches. The base used in the reactions may preferably be organic bases, more preferably pyridine, substituted pyridine, piperidine, or C₁-C₄ aliphatic amine. The reaction solvent may preferably be C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof.

In some embodiments, in the reaction of protecting 5-hydroxyl of compound IV with a methanesulfonyl group under a basic condition to obtain compound V, the reaction temperature may be from -30 °C to 60 °C, the amount of methanesulfonyl chloride may be from 0.6 to 3 times of that of compound III (in molar ratio). The base used in the reactions may preferably be organic bases, more preferably pyridine, substituted pyridine, piperidine, or C₁-C₄ aliphatic amine. The reaction solvent may preferably be C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof. A crude product may be refined by recrystallization.

In which, the synthesis of compound III can be referred to the method of J. Org. Chem. 2003, 68, 7559.

In the context of the present invention, Bn represents a benzyl group, Ms represents a methanesulfonyl group, Bz represents a benzoyl group.

One object of the present invention is to provide a method for preparing 3-O-benzyl-1,2-O-isopropylidene-α-L-idofuranose, which includes the following steps: 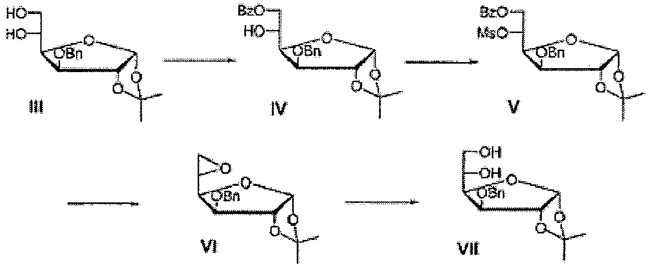
(1) by taking advantage of steric hindrance, 6-hydroxyl of compound III was firstly protected by a benzoyl group under a basic condition to obtain compound IV, and then 5-hydroxyl of compound IV was protected by methanesulfonyl group under a basic condition to obtain compound V;
(2) the cyclization of compound V is conducted under a basic condition to obtain 5,6-epoxy compound VI with inversion of configuration at 5-position;
(3) the selective ring-opening of compound VI is conducted under a basic condition to obtain compound VII.

In some embodiments, in step (1), during the reaction of selectively protecting 6-hydroxyl of compound III with a benzoyl group under a basic condition to obtain compound IV, wherein the reaction temperature may be from -30 °C to +60 °C, and the amount of benzoyl chloride may be from 0.6 to 2.0 times of that of compound III (in molar ratio), which was added in batches. The base used in the reactions may preferably be organic bases, more preferably pyridine, substituted pyridine, piperidine, or C₁-C₄ aliphatic amine. The reaction solvent may preferably be C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof. In the reaction of protecting 5-hydroxyl of compound IV with a methanesulfonyl group under a basic condition to obtain compound V, the reaction temperature may be from -30 °C to 60 °C, and the amount of methanesulfonyl chloride may be from 0.6 to 3 times of that of compound III (in molar ratio). The base used in the reactions may preferably be organic bases, more preferably pyridine, substituted pyridine, piperidine, or C₁-C₄ aliphatic amine. The reaction solvent may preferably be C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof. A crude product may be refined by recrystallization.

In some embodiments, in step (2), during the reaction of compound V under a basic condition of forming 5,6-epoxy compound VI with inversion of configuration at 5-position, the reaction temperature may be from -30 °C to +60 °C. The amount of base may be from 1 to 10 times of that of compound V (in molar ratio). The base may preferably be inorganic bases, and organic bases, wherein the inorganic base may preferably be potassium hydroxide, sodium hydroxide, lithium hydroxide, magnesium hydroxide, calcium hydroxide, barium hydroxide, and wherein the organic base may preferably be pyridine, triethylamine, sodium C₁-C₄ lower alcoholate or potassium C₁-C₄ lower alcoholate, wherein the preferred sodium C₁-C₄ lower alcoholate or potassium C₁-C₄ lower alcoholate is sodium methoxide, sodium ethoxide, sodium tert-butoxide, potassium tert-butoxide. The reaction solvent may preferably be water, methanol, ethanol, propanol, t-butanol, tetrahydrofuran, dioxane, acetonitrile, N,N-dimethyl formamide, dimethyl sulfoxide, dichloromethane, or any mixtures thereof. The reaction solution may be conducted into the next step directly without any post-treatment, or the crude product after post-treatments may be used directly in the next step.

In some embodiments, in step (3), during the ring-opening reaction of compound VI under a basic condition to obtain compound VII, the reaction temperature may be from -30 °C to +100 °C. The amount of base may be from 1 to 10 times of that of compound V (in molar ratio). The base may preferably be inorganic bases, wherein the inorganic base may preferably be potassium hydroxide, sodium hydroxide, lithium hydroxide, magnesium hydroxide, calcium hydroxide, barium hydroxide. The reaction solvent may preferably be water, methanol, ethanol, propanol, t-butanol, tetrahydrofuran, dioxane, acetonitrile, N,N-dimethyl formamide, dimethyl sulfoxide, or any mixtures thereof. A crude product may be refined by recrystallization.

In which, the synthesis of compound III in step (1) can be referred to the method of J. Org. Chem. 2003, 68, 7559.

Comparing with the prior art, the advantages of the present invention lie in:
Throughout the prior art of synthesizing the derivatives of idose, as for the route of inversion of configuration of the 5-hydroxyl group by hydroboration-oxidation reaction on the 5,6-exocyclic double bond of the glucose, it is long, complicated to operate, and the yield is low, as well as the purification is difficult. As for the method of inversion of configuration by isomerization through a free radical reaction of 5-bromo-glucuronic acid, it is inefficient as well as some expensive and highly toxic reagents are used. As for the method of constructing chirality at 5-position by using ions such as cyano, (PhS)₃CLi, etc. to conduct an asymmetric addiction to an aldehyde, it is long route and needs harsh reaction conditions, and the stereoselectivity of this route is not satisfactory and the product thereof is difficult to purify. As for the method of inversion of configuration of the 5-hydroxyl group by nucleophilic substitution on the trifluoromethanesulfonate or methane sulfonate of glucose or glucuronic acid , its yield is low, while the side-reactions are serious as well as the purification thereof is difficult. As for the method of inversion of configuration of the 5-hydroxyl group by utilizing acidic ring-opening the epoxy group, no effective, easy, suitable method for a large scale industrial production on the ring-opening epoxy after inversion of configuration, and the purification of products is difficult. The advantages of the present invention are as follows: a short process route, a high yield, mild reaction conditions, simple operation procession, no expensive and highly toxic agents, low cost, as well as the purification of intermediates and products can be performed by recrystallization, and the method is suitable for a large-scale industrial production.

### Detailed Embodiments

### Example 1

### Preparation of 3-O-benzyl-1,2-O-isopropylidene-α-D-glucofuranose III

### Step a: preparing 3-O-benzyl-1,2:5,6-di-O-isopropylidene-α-D-glucofuranose

Under argon atmosphere, 1 L of tetrahydrofuran, 64 g of sodium hydride (60%) were successively added into a 5 L four-necked reaction flask, then the reactants were cooled down to 0∼5 °C with an ice-water bath. A mixture solution of 315 g of 1,2:5,6-di-O-isopropylidene-α-D-glucofuranose and 1 L of tetrahydrofuran was added dropwise. The temperature was maintained at 0∼10 °C. The reaction was conducted at about 0 °C for 5 hours. 220 mL Benzyl bromide was added dropwise, and the temperature was maintained at 0∼10 °C, and then 20 g of tetrabutylammonium bromide was added. The ice-water bath was removed, and the temperature climbed up to room temperature naturally. Continue stirring till the reaction was completed. A small amount of methanol was added dropwise to quench the reaction. The solvent was concentrated and removed under a reduced pressure. The reaction was dissolved by adding ethyl acetate, and washed 3 times with water, dried over anhydrous sodium sulfate, and concentrated to provide 540 g of crude product which was used in the next step directly.

### Step b: preparing 3-O-benzyl-1,2-O-isopropylidene-α-D-glucofuranose III

540 g Crude product of 3-O-benzyl-1,2:5,6-di-O-isopropylidene-α-D-glucofuranose obtained from step a was weighted and put into a 3 L single-necked flask. 1.8 L of 60% aqueous acetic acid solution was injected, and the reaction solution was stirred at room temperature overnight. The reaction was monitored by TLC till it was completed. 1.8 L Water was added, and the reactants were washed for three times with petroleum ether to remove the low polarity impurities, and the aqueous phase was neutralized with sodium bicarbonate solid, extracted with ethyl acetate for three times, dried over anhydrous sodium sulfate, and concentrated to provide 342 g of crude product (HPLC, purity: 91%). The yield of the two-step reaction is 91%. No further purification is required. The next step was conducted directly. A small amount of sample after column chromatography purification was analyzed by MS, ¹H NMR and ¹³C NMR.

ESI-MS (m/z): 333.1 [M+Na]⁺; ¹H NMR (400 MHz, CDCl₃ ): δ 7.32-7.24 (m, 5 H), 5.88 (d, J = 3.2 Hz, 1 H), 4.61 (m, 2 H), 4.56 (s, 1 H), 4.12-4.08 (m, 2 H), 4.00 (m, 1 H), 3.80-3.60 (m, 2 H), 3.27 (br, 2 H), 1.45 (s, 3 H), 1.27 (s, 3 H); ¹³C NMR (100 MHz, CDCl₃): δ 137.46, 128.54, 127.98, 127.72, 111.71, 106.09, 82.19, 81.88, 79.96, 77.52, 76.88, 72.23, 68.96, 64.26, 26.88, 26.17.

### Example 2

### Preparation of 6-O-benzoyl-3-O-benzyl-1,2-O-isopropylidene-α-D-glucofuranose IV and 6-O-benzoyl-3-O-benzyl-1,2-O-isopropylidene-5-O-methanesulfonyl-α-D-glucofuranose V

96.5 g Compound III was dissolved in 500 mL of pyridine, and cooled to -10 °C with an ice salt bath, and a solution of 36.89 mL of benzoyl chloride in 40 mL of dichloromethane was dropwise added in batches. The reaction temperature was maintained at -10∼0 °C. After the reaction was completed, the reaction mixture was used directly in the next step without separation and purification.

The reaction was cooled to 0 °C with an ice-water bath. 26 mL Methanesulfonyl chloride was slowly added into the above reaction mixture in drops, and the reaction solution was stirred overnight. After the reaction was completed, the reaction solution was put into 2 L of 55∼60 °C warm water. A white crystal was precipitated after cooling the reaction solution, filtered and dried, and then recrystallized by ethanol. The solid was dried to provide 114.8 g of compound V. The yield of two steps is 75%.

The data of MS, ¹H NMR and ¹³C NMR measurements of compound V are as follows:
ESI-MS (m/z): 493.1 [M+H]⁺; ¹H NMR (400 MHz, CDCl₃): δ 8.07 (d, J = 7.2 Hz, 2 H), 7.56 (t, J = 7.6 Hz, 1 H), 7.28-7.43 (m, 7 H), 5.91 (d, J = 3.6 Hz, 1 H), 5.41 (m, 1 H), 4.92 (d, J = 12.4 Hz, 1 H), 4.70 (d, J = 10.8 Hz, 1 H), 4.62 (s, 1 H), 4.60 (d, J = 7.6 Hz, 1 H), 4.50-4.40 (m, 2 H), 4.14 (d, J =2.8 Hz, 1 H), 2.98 (s, 3 H), 1.50 (s, 3 H), 1.31 (s, 3 H); ¹³C NMR (100 MHz, CDCl₃): δ 165.57, 137.16, 133.31, 129.75, 129.69, 128.53, 128.41, 128.36, 128.12, 128.08, 112.30, 106.41 , 81.58, 81.15, 78.29, 77.46, 76.14, 76.82, 75.37, 72.37, 64.10, 39.12, 26.88, 26.32.

### Example 3

### Preparation of 5,6-epoxy-3-O-benzyl-1,2-O-isopropylidene-α-L-idofuranose VI

50 g Compound V was dissolved in 300 mL of dioxane, and at room temperature, a solution of 29 g of potassium hydroxide in 100 mL of water was slowly added. After the completion of dropping, the reaction solution was stirred at room temperature till the reaction was completed. The reaction mixture was cooled with an ice-water bath, and hydrochloric acid was slowly added to neutralize the solution till neutralization. The solvents having low boiling points were removed under a reduced pressure. The resulted mixture was extracted with ethyl acetate. The organic phase was combined, and successively washed with a saturated sodium bicarbonate solution, a saturated sodium chloride solution, then dried over anhydrous sodium sulfate. 30.5 g Crude compound VI was obtained after concentration. The crude product was used directly in the next step without further purification.

### Example 4

Preparation of 5,6-epoxy-3-O-benzyl-1,2-O-isopropylidene-α-L-idofuranose VI 20 g Compound V was dissolved in the mixture solvent of 200 mL of dichloromethane and 100 mL of tert-butanol, and cooled in an ice-water bath. 10.5 g Potassium tert-butoxide was slowly added into the reaction solution. After completion of adding, the reaction was stirred at room temperature till the reaction was completed. The reaction mixture was cooled with an ice-water bath, and hydrochloric acid was slowly added to neutralize the solution till neutralization. The obtained mixture was extracted with ethyl acetate. The organic phase was combined, and successively washed with a saturated sodium bicarbonate solution, a saturated sodium chloride solution, then dried over anhydrous sodium sulfate, and purified on a chromatography column to obtain 11.4 g of compound VI after concentration.

The data of MS, ¹H NMR and ¹³C NMR measurements of compound VI are as follows:
ESI-MS (m/z): 331.0 [M+K]⁺; ¹H NMR (400 MHz, CDCl₃): δ 7.37-7.30 (m, 5 H), 6.00 (d, J = 4.0 Hz, 1 H), 4.74 (d, J = 12.4 Hz, 1 H), 4.64 (d, J = 4.0 Hz, 1 H), 4.51 (d, J = 12.4 Hz, 1 H), 3.97 (d, J = 3.2 Hz, 1 H), 3.80 (dd, J₁ = 6.0 Hz, J₂ = 3.2 Hz, 1 H), 3.27 (m, 1 H), 2.75 (t, J = 4.4 Hz, 1 H), 2.53(m, 1 H), 1.45 (s, 3 H), 1.32 (s,3 H); ¹³C NMR (100 MHz, CDCl₃): δ 137.16, 128.39, 127.92, 127.56, 111.77, 105.33, 82.58, 82.29, 81.99, 71.80, 50.05, 43.02, 26.73, 26.20.

### Example 5

### Preparation of 3-O-benzyl-1,2-O-isopropylidene-α-L-idofuranose VII

30.5 g Crude compound VI from Example 3 was dissolved in 200 mL of dimethyl sulfoxide, and then a solution of 30.3 g of potassium hydroxide in 100 mL of water was slowly added. After the completion of dropping, the mixture was heated to 60-70 °C to react till the reaction was completed. The reaction was diluted with water, and extracted with dichloromethane, and successively washed with a saturated sodium chloride solution, then dried over anhydrous sodium sulfate, and then the solvents were removed under a reduced pressure. The crude product was recrystallized and dried to provide 25.2 g of compound VII.

The data of MS, ¹H NMR and ¹³C NMR measurements of compound VII are as follows:
ESI-MS (m/z): 333.1 [M+Na]⁺; ¹H NMR (400 MHz, CDCl₃): δ 7.34-7.27 (m, 5 H), 5.95 (d, J = 3.2 Hz, 1 H), 4.63-4.44 (m, 3 H), 4.20 (m, 1 H), 4.04 (m, 1 H), 3.97 (d, J = 3.2 Hz, 1 H), 3.65-3.50 (m, 2 H), 3.51 (s, 2 H), 1.45 (s, 3 H), 1.29 (s, 3 H); ¹³C NMR (100 MHz, CDCl₃): δ 138.88, 128.66, 128.10, 127.83, 111.76, 104.72, 82.40, 82.17, 80.17, 77.69, 76.98, 71.72, 70.60, 63.22, 26.70, 26.26.

### Example 6

### Preparation of 5,6-epoxy-3-O-benzyl-1,2-O-isopropylidene-α-L-idofuranose VI and 3-O-benzyl-1,2-O-isopropylidene-α-L-idofuranose VII

114.8 g Compound V was dissolved in 500 mL of N,N-dimethyl formamide, and at room temperature, a solution of 65.37 g of potassium hydroxide in 262 mL of water was slowly added. The clear reaction solution was turned into brownish black till the reaction was completed. The reaction product was used directly in the next step without separation and purification.

The above reaction solution was directly heated to 60-70 °C to continue the reaction till it was completed. The reaction solution was diluted by water, and extracted with dichloromethane, washed with a saturated sodium chloride solution, then dried over anhydrous sodium sulfate. The solvents were removed under a reduced pressure. The crude product was recrystallized and dried to provide 55.61 g of compound VII.

The data of MS, ¹H NMR and ¹³C NMR measurements of compound VII are as follows:
ESI-MS (m/z): 333.1 [M+Na]⁺; ¹H NMR (400 MHz, CDCl₃): δ 7.34-7.27 (m, 5 H), 5.95 (d, J = 3.2 Hz, 1 H), 4.63-4.44 (m, 3 H), 4.20 (m, 1 H), 4.04 (m, 1 H), 3.97 (d, J = 3.2 Hz, 1 H,), 3.65-3.50 (m, 2 H), 3.51 (s, 2 H), 1.45 (s, 3 H), 1.29 (s, 3 H); ¹³C NMR (100 MHz, CDCl₃): δ 138.88, 128.66, 128.10, 127.83, 111.76, 104.72, 82.40, 82.17, 80.17, 77.69, 76.98, 71.72, 70.60, 63.22, 26.70, 26.26.

### Example 7

### Preparation of 5,6-epoxy-3-O-benzyl-1,2-O-isopropylidene-α-L-idofuranose VI and 3-O-benzyl-1,2-O-isopropylidene-α-L-idofuranose VII

80 g Compound V was dissolved in 350 mL of dimethyl sulfoxide, and at room temperature, a solution of 46 g of potassium hydroxide in 180 mL of water was slowly added. The reaction was stirred at room temperature till the reaction was completed. The reaction product was used directly in the next step without separation and purification.

The above reaction solution was directly heated to 60-70 °C to continue the reaction till it was completed. The reaction was diluted by water, and extracted with dichloromethane, and washed with a saturated sodium chloride solution, then dried over anhydrous sodium sulfate. The solvents were removed under a reduced pressure. The crude product was recrystallized and dried to provide 41 g of compound VII.

The data of MS, ¹H NMR and ¹³C NMR measurements of compound VII are as follows:
ESI-MS (m/z): 333.1 [M+Na]⁺; ¹H NMR (400 MHz, CDCl₃): δ 7.34-7.27 (m, 5 H), 5.95 (d, J = 3.2 Hz, 1 H), 4.63-4.44 (m, 3 H), 4.20 (m, 1 H), 4.04 (m, 1 H), 3.97 (d, J = 3.2 Hz, 1 H,), 3.65-3.50 (m, 2 H), 3.51 (s, 2 H), 1.45 (s, 3 H), 1.29 (s, 3 H); ¹³C NMR (100 MHz, CDCl₃): δ 138.88, 128.66, 128.10, 127.83, 111.76, 104.72, 82.40, 82.17, 80.17, 77.69, 76.98, 71.72, 70.60, 63.22, 26.70, 26.26.

### Example 8

### Preparation of 5,6-epoxy-3-O-benzyl-1,2-O-isopropylidene-α-L-idofuranose VI and 3-O-benzyl-1,2-O-isopropylidene-α-L-idofuranose VII

82 g Compound V was dissolved in 350 mL of dioxane, and at room temperature, a solution of 46 g of potassium hydroxide in 180 mL of water was slowly added. The reaction was stirred at room temperature till it was completed. The reaction product was used directly in the next step without separation and purification.

The above reaction solution was directly heated to 60-70 °C to continue the reaction till it was completed. The reaction solution was diluted by adding water, and extracted with dichloromethane, and washed with a saturated sodium chloride solution, then dried over anhydrous sodium sulfate. The solvents were removed under a reduced pressure. The crude product was recrystallized and dried to provide 38 g of compound VII.

The data of MS, ¹H NMR and ¹³C NMR measurements of compound VII are as follows:
ESI-MS (m/z): 333.1 [M+Na]⁺; ¹H NMR (400 MHz, CDCl₃): δ 7.34-7.27 (m, 5 H), 5.95 (d, J = 3.2 Hz, 1 H), 4.63-4.44 (m, 3 H), 4.20 (m, 1 H), 4.04 (m, 1 H), 3.97 (d, J = 3.2 Hz, 1 H), 3.65-3.50 (m, 2 H), 3.51 (s, 2 H), 1.45 (s, 3 H), 1.29 (s, 3 H); ¹³C NMR (100 MHz, CDCl₃): δ 138.88, 128.66, 128.10, 127.83, 111.76, 104.72, 82.40, 82.17, 80.17, 77.69, 76.98, 71.72, 70.60, 63.22, 26.70, 26.26.

## Claims

1. A method for preparing 3-O-benzyl-1,2-O-isopropylidene-α-L-idofuranose VII, wherein conducting a selective ring-opening of compound VI under a basic condition to obtain compound VII:

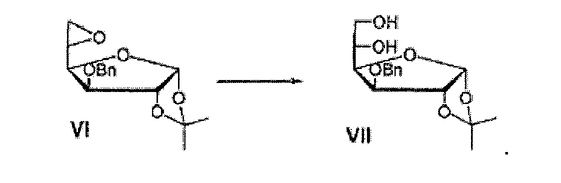

2. The method according to claim 1, wherein the reaction temperature is from -30 °C to +100 °C, the base used in the reaction is selected from inorganic bases, and the inorganic base preferably is potassium hydroxide, sodium hydroxide, lithium hydroxide, magnesium hydroxide, calcium hydroxide, barium hydroxide.

3. The method according to claim 1, wherein the reaction solvent is selected from water, methanol, ethanol, propanol, t-butanol, tetrahydrofuran, dioxane, acetonitrile, N,N-dimethyl formamide, dimethyl sulfoxide, or any mixtures thereof.

4. The method according to claim 1, wherein compound V is undergone a cyclization reaction under a basic condition to obtain 5,6-epoxy compound VI with inversion of configuration at 5-position:

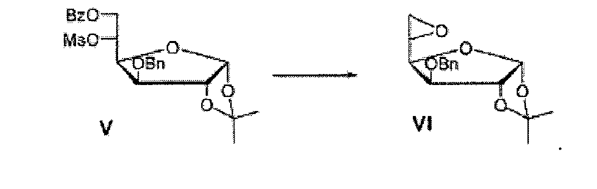

5. The method according to claim 4, wherein the base used in the cyclization reaction is selected from inorganic bases, organic bases, wherein the inorganic base preferably is potassium hydroxide, sodium hydroxide, lithium hydroxide, magnesium hydroxide, calcium hydroxide, barium hydroxide, and wherein the organic base preferably is pyridine, triethylamine, sodium C₁-C₄ lower alcoholate or potassium C₁-C₄ lower alcoholate, wherein the sodium C₁-C₄ lower alcoholate or potassium C₁-C₄ lower alcoholate preferably is sodium methoxide, sodium ethoxide, sodium tert-butoxide, potassium tert-butoxide.

6. The method according to claim 4, wherein the reaction solvent used in the cyclization reaction is selected from water, methanol, ethanol, propanol, t-butanol, tetrahydrofuran, dioxane, acetonitrile, N,N-dimethyl formamide, dimethyl sulfoxide, dichloromethane, or any mixtures thereof.

7. The method according to claim 4, wherein by taking advantage of steric hindrance, 6-hydroxyl of compound III is firstly protected by a benzoyl group under a basic condition to obtain compound IV, and then 5-hydroxyl of compound IV is protected by methanesulfonyl group under a basic condition to obtain compound V:

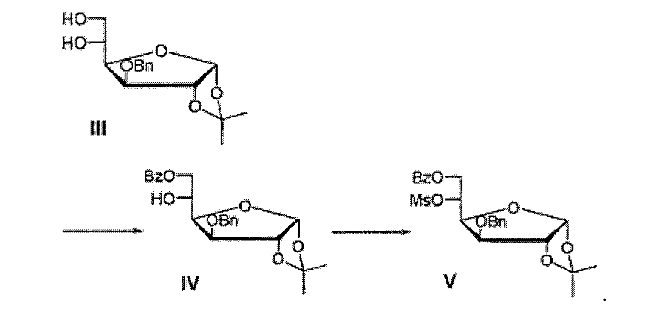

8. The method according to claim 7, wherein during the protection by the benzoyl group, the reaction is conducted at the temperature of -30 °C - 60 °C, the base used in the reactions is selected from organic bases, in which the organic base preferably is pyridine, substituted pyridine, piperidine, or C₁-C₄ aliphatic amine; the reaction solvent is selected from C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof.

9. The method according to claim 7, wherein during the protection by the methanesulfonyl group, the reaction temperature is conducted at the temperature of -30 °C - 60 °C, the base used in the reactions is selected from organic bases, in which the organic base preferably is pyridine, substituted pyridine, piperidine, or C₁-C₄ aliphatic amine; the reaction solvent is selected from C₁-C₆ monohalogenated alkanes or polyhalogenated alkanes, tetrahydrofuran, acetonitrile, pyridine, dioxane, N,N-dimethyl formamide, dimethyl sulfoxide, or any mixtures thereof.

10. The method according to claim 7, wherein during the protection by the methanesulfonyl group, the reaction solution is poured into water, and cooled down to separate out crystals, and then to recrystallize.

11. Use of compound VI in the preparation of compound VII
